# EUROPEAN PATENT APPLICATION

(11) **EP 3 985 097 A1**
(43) Date of publication of application: **20.04.2022**
(21) Application number: 20201696.0
(22) Date of filing: 14.10.2020
(51) Int. Cl.: C12M 1/36, C12M 1/00

(54) **A METHOD AND SYSTEM FOR CONFIGURING AND/OR SETUP OF A DOWNSTREAM PROCESS FOR PROCESSING A BIOMASS**

(71) Applicant: Sartorius Stedim Biotech GmbH, 37079 Göttingen (DE)
(72) Inventor: LEUPOLD, Marco, 37075 Göttingen (DE); ESPACHS, Alexandre, 37079 Göttingen (DE); HUSEMANN, Bernward, 37079 Göttingen (DE); RODENBERG, Michael, 37574 Einbeck (DE); MATUSZCZYK, Jens, 37079 Göttingen (DE); KAMPMANN, Markus, 44149 Dortmund (DE)
(74) Representative: Müller-Boré & Partner Patentanwälte PartG mbB

(57) **Abstract**

The invention relates to a computer-implemented method for configuring and/or setup and/or controlling of a downstream process for processing a biomass, comprising:
receiving at least one measurement value of at least one feature of an upstream process by which the biomass to be processed in the downstream process has been obtained;
determining at least one downstream process parameter based on the received at least one measurement value.

The invention further relates to a computer-program product and a system for configuring and/or setup and/or controlling of a downstream process, a method and a system for processing a biomass.

## Description

The technical field of the present application is methods and systems for configuring, setup and/or control of a downstream process for biomass processing. In particular an aspect of the application relates to an interconnection between upstream and downstream processes for biomass processing.

A process for processing a biomass to obtain a product (bioprocess) is a process using cells and/or their components to obtain desired products. A bioprocess typically comprises a plurality of different stages or operations, for example cell growth stage, cell harvesting stage, media preparation stage, separation stage, chromatography stage, filtering stage, stirring stage, etc.

Generally, a bioprocess may be divided in an upstream process and a downstream process. The upstream process (upstream part of the bioprocess) relates to the first part of a bioprocess, in which the cell culture, microbes, etc. is/are grown. An upstream process typically involves a plurality of steps or stages. For example, an upstream process may comprise culture inoculation, culture growth, fermentation, etc. A downstream process (downstream part of the bioprocess) typically involves processing the biomass produced by the upstream process to obtain a final product meeting certain quality and purity requirements. A downstream process typically involves a plurality of stages. For example, a downstream process may comprise a primary recovery or clarification stage at which cells and/or debris are removed, separation stage (e.g. by centrifugation), filter stage, chromatography stage, buffer exchange stage, material hold stage, viral inactivation stage, purification stage, etc. Each of the stages of the upstream and downstream process may comprise a plurality of sub-stages. Each individual stage or operation has one or more process parameters or settings that need to be configured, setup and/or controlled to obtain the required final product.

There are various challenges involved in the configuring or setup up and controlling the different stages of a complex bioprocess. An object of the present invention is to provide an improved methods and systems for configuring, setup and/or control of a bioprocess process.

According to an aspect of the present disclosure, there is provided a computer-implemented method for configuring and/or setup of a downstream process for processing a biomass, comprising:
receiving at least one measurement value of at least one feature of an upstream process by which the biomass to be processed in the downstream process has been obtained;
determining at least one downstream process parameter based on the received at least one measurement value.

The downstream process is configured and/or setup based on the determined at least one downstream process parameter. Thus, the downstream process is carried out in accordance with the at least determined downstream process parameter.

According to a second aspect of the present disclosure, there is provided a computer-implemented method for controlling of a downstream process for processing a biomass, comprising:
receiving at least one measurement value of at least one feature of an upstream process by which the biomass to be processed in the downstream process has been obtained;
determining at least one downstream process parameter based on the received at least one measurement value;
controlling at least one process stage or process operation of the downstream process based on the determined at least one downstream process parameter.

Examples of the biomass to be processed include bacteria, yeasts, molds, animal cells, plant cells, etc. Further ingredients may include chemical compounds, proteins (such as enzymes), various additives, etc.

The downstream processing of the biomass results in a biopharmaceutical product. Some non-limiting examples of such products include recombinant and non-recombinant proteins, vaccines, gene vectors, DNA, RNA, antibiotics, secondary metabolites, growth factors, cells for cell therapy or regenerative medicine, half-synthesized products (e.g. artificial organs). Various production systems may be used to facilitate the process, e.g. cell based systems such as animal cells (e.g. CHO, HEK, PerC6, VERO, MDCK), insect cells (e.g. SF9, SF21), microorganisms (e.g. E. coli, S. cerevisiae, P. pastoris, etc.), algae, plant cells, cell free expression systems (cell extracts, recombinant ribosomal systems, etc.), primary cells, stem cells, native and gene manipulated patient specific cells, matrix based cell systems.

As mentioned above, an upstream process is a bioprocess by which a cell culture, microbial culture, etc. is grown, thereby producing a biomass that is further processed in a downstream process to obtain a final product meeting the requirements for purity, quality, etc. Typically, each of the upstream and downstream process comprises a plurality of stages, each stage being configured to perform a certain process operation or a group of process operations. For example, an upstream process may comprise one or more of the following stages or unit operations: culture inoculation, culture growth, fermentation, etc. The downstream process may comprise one or more of the following stages or unit operations primary recovery or clarification stage at which cells and/or debris are removed, separation stage (e.g. by centrifugation), filter stage, chromatography stage, buffer exchange, material hold stage, viral inactivation stage, purification stage, etc. Each of the stages of the upstream and downstream process may comprise a plurality of sub-stages. Each individual stage or operation may have one or more process parameters or settings that need to be configured, setup and/or controlled to obtain the required final product.

Examples of end products of the downstream process include but are not limited to recombinant or non-recombinant proteins, vaccines, gene vectors, DNA, RNA, antibiotics, secondary metabolites, growth factors, etc.

At least one feature (upstream feature) of the upstream process by which the biomass to be processed in the downstream process has been obtained may be measured and the respective measurement value or values made available to the downstream process. The at least one upstream feature characterizes the cells, contaminants, a product and/or a process parameter of the upstream process. The at least one upstream feature may be a feature that characterizes any stage/process operation of the upstream process.

Measurements of the at least one upstream process feature may be performed online or offline. In the context of the present description the term "online" includes also inline and atline measurements. Atline measurements may require more time and more manual intervention than inline measurements. However, atline measurements might not involve the time, the level of analysis and manual intervention required for offline measurements.

Online measurements (such as inline measurements) typically do not require removal of a sample of the product, although in some cases online measurements (such as atline measurements) may also involve removal or diversion of a sample of the product to be produced. Online measurements may be obtained using probes, sensors, or measuring devices placed in the processed product and/or the container holding the processed product (such a bioreactor). Online measurements may be carried out at specified intervals throughout the process and may correspond to process parameters that have a defined confidence interval. Examples of online measurements include pH, conductivity, temperature, culture volume, antifoam concentration, cell density, cell turbidity, cell viability, flow sensor parameter, including sensor quality, spectroscopy data, e.g. indicative of the total protein concentration, metabolite and/or medium concentration, pressure value of a tube and/or bioreactor, etc.

Offline measurements may require a sample of the processed product to be diverted for in-depth analysis. In some cases, offline measurements may be performed once for a batch. Examples of offline measurements include cell viability, total protein concentration obtained by offline measurement or offline sample, lactate dehydrogenase or other enzyme activity, upstream product quality, e.g. of a monoclonal antibody, upstream product aggregation level, product concentration, DNA/HCP concentration, titer, etc.

The measurement value(s) of the at least one upstream feature may be made available to the downstream process. For example, the measured value(s) may be transmitted to a receiving device involved in the downstream process by means of a wireless transmission, via computer network (e.g. over the Internet) or by any other suitable communication means.

Based on the measurement value(s) of the at least one upstream feature, one or more settings, i.e. one or more parameters of the downstream process are determined, for example by a suitable programmed computer system comprising one or more processing units (processors). The computer system may be a part of a system for configuring and/or setup and/or controlling a downstream process (downstream process setup and/or control system).

The determined downstream process parameter(s) may be stored in a database or other suitable data storage unit. The database may further store additional process parameters or data, such as for example (functional) relations or rules linking the at least one upstream feature and its values and at least one downstream parameter or setting. The stored process parameter(s), optionally together with additional parameters or data, may be used to automatically setup, configure and/or control at least one downstream process stage or process operation. The database may be accordingly accessible over a suitable computer network by the respective devices and/or control units.

The database may be implemented as a cloud database, i.e. a database that runs on a cloud computing platform. In other words, the database may be accessible over the Internet via a provider that makes shared processing resources and data available to computers and other devices on demand. The database may be implemented using a virtual machine image or a database service. The database may use an SQL based or NoSQL data model.

The database may provide access control such that some of the process parameters are accessible by a plurality of users of the database or respective control devices and some process parameters are private and only accessible by a limited number of users or one particular user or respective control devices.

In the context of the present description, the term "determining" encompasses any of the following operations: calculating, setting, changing and/or adjusting of at least one downstream process parameter, i.e. of the settings relating to at least one process parameter of the downstream process. The term "receiving" encompasses any of the following operations: obtaining the respective data (measurement value(s) of at least one upstream feature), receiving wirelessly transmitted data, receiving data transmitted over computer networks such as Internet, WLAN, etc., reading data from a permanent or temporary storage unit (such as example database) or receiving or obtaining data by any suitable means. The data may be received via a suitable interface or interfaces of a computer system. Controlling of at least one process stage or process operation (such as filtering, chromatography, pumping, etc.) may comprise controlling of at least one process equipment and/or control device associated with the respective process stage or process operations based on determined downstream process parameters. The at least one control device may be a part of a control system (i.e. a downstream process control system). The controlling may be carried out while the downstream process and more specifically the respective process stage or process operation is performed using respective process equipment.

According to the first aspect described above, the downstream process is automatically setup, configured and/or controlled based on measurements of at least one feature of the upstream process by which the biomass to be processed in the downstream process is obtained. Advantages of the proposed method for controlling a downstream process include reducing the number of labor-intensive and error prone manual operations and thus the overall error rate, optimizing the processing time, simplifying the downstream setup and control process, improving the quality of the output products and reducing waste. Further, it is possible to optimally synchronize the upstream and downstream process stages, which enables reduction of idle, non-productive time and increasing the process efficiency. Still further, the logistic and stock management may be improved.

Various features or characteristics of the upstream process may be measured (for example automatically) and the measured values made available to the downstream process.

For example, the at least one upstream process feature may include a viable cell density, biomass, capacitance, cell viability and/or turbidity. Based on the measured upstream values, at least one downstream parameter setting may be determined. The determining of the at least one downstream process parameter may for example comprise:
selecting a clarification operation;
determining, based on the received at least one measurement value of the viable cell density, biomass, capacitance and/or cell viability, at least one process parameter of the selected clarification operation and/or determining a biomass dilution parameter.

The selecting of a clarification operation may include selecting between a filter operation and a centrifugal operation, wherein:
if a filter operation is selected, the at least one process parameter may include at least one of the following parameters: a type of the filter operation, number of filters, filter area, pressure, flow rate, amount of diatomaceous earth and process time;
if the centrifugal operation is selected, the at least one process parameter may include the process time, process volume and the centrifugal force.

For example, the measured value of the viable cell density (VCD) of the upstream process may be multiplied with the process volume, in order to determine the overall biomass amount to be processed in the downstream process. Based on the determined biomass amount, the amount of biomass that needs to be separated or clarified may be determined. The amount of biomass that needs to be separated or clarified may also be determined based on the measured turbidity or measured biomass.

Depending on the biomass that needs to be separated or clarified, a suitable separation method and/or system for the cell separation stage may be determined. The separation method and the respective system may be, for example, a filtering method/system and centrifugal separation. Further, among the filtering methods, different types of filtering may be selected such as Deep Filtration, Dynamic Body Feed Filtration, etc.

The (functional) relationship or rule linking the biomass that needs to be separated and respective clarification operation settings (such as the separation method and/or system) may be stored in a database or other suitable storage media and used to configure, setup and/or control the downstream process. For example, with the increasing amount of the biomass that needs to be separated, the following separation methods and/or systems may be selected in this order: Deep filtration, Dynamic Body Feed Filtration, or Centrifugal separation. In addition, other separation settings, such as the number of the filters, the filter area and/or the process time may be determined. Still further, the level of dilution of the biomass to be processed may be determined, in order to facilitate to separation process.

In addition or alternatively, the downstream clarification operation settings (such as for example, the g-force of the centrifugation stage, the process time, etc.) may be determined based on the cell viability. For example, lower g-force of the centrifugal stage/operation may be selected for cells that should retain high target viability as compared to the g-force of cells that are damaged or apoptotic. In addition or alternatively, the downstream clarification settings may be determined based on the targeted degree of sedimentation. For example, the higher the targeted degree of sedimentation (independent of the cell viability), the higher the g-force and/or the process time of the centrifugation operation.

In an example, the at least one upstream process feature may include titer. The determining of the at least one downstream process parameter may comprise determining, based on the received measurement value of the titer, at least one process parameter of a chromatography operation, said at least one process parameter including one or more of a dimensioning, loading volume, cycle time, flow rate, number of chromatography columns, column exchange sequence, loading scenario and/or sequence. The (functional) relationships or rules linking the measured upstream titer and the at least one process parameter of a chromatography operation may be stored in a database or other suitable storage unit and used to configure, setup and/or control the downstream process.

A high titer multiplied by the process volume results in a large quantity of product. Generally, the higher the titer, the smaller the volume that is to be loaded in/over a chromatography column in a loading step. Accordingly, with the increase of the upstream titer, in a downstream process, a larger size and/or higher number of chromatography columns and/or a higher number of cycles may be set/configured.

For example, in a batch process, the measured value of the titer may be multiplied by the process volume to determine the overall amount of the product (biomass) to be processed in the downstream process, for example to be purified by protein A chromatography. Based on the determined overall amount of biomass, the chromatography settings (such as the dimensioning or size of the chromatographic column(s), loading volume, etc.) required for processing the biomass within a predetermined time may be determined and set accordingly. Alternatively, for a given size or dimensions of the chromatography column(s) the processing time (e.g. the number of cycles) may be determined and set accordingly. Further, the amount of puffer may be determined and set accordingly.

In case of a continuous process, based on the titer and the volume flow, the number of chromatography columns necessary to ensure the continuous processing of the flow of biomass from the upstream process to the downstream process may be determined. Further or alternatively, based on the titer, the number of cycles and/or the timing of the chromatography column(s) exchange (e.g. because of the column's aging after a certain number of cycles) may be determined. Further, based on the titer, at least one recipe of the downstream process may be determined (e.g. at least one recipe according to ISA 88). The determination of the recipe may comprise a new recipe's generation or a change or adjustment of an existing recipe. The generation, change or adjustment of a recipe my for example comprise the determination of a volume flow, the loading volume and/or other parameters. The term "recipe" as used in the present application relates to the necessary set of information that uniquely defines the production requirements for a specific product or operational task (see also the definition of the term "recipe" in ANSI/ISA-88.00.01-201o "Batch Control Part 1: Models and Terminology"). Chapter 6, point 6.1, page 55).

In an example, the at least one upstream process feature may include a host cell protein (HCP) and/or DNA concentration. The determining may comprise determining of the at least one process parameter of an anion or a cation exchange chromatography operation, said at least one process parameter including one or more of a volume of the chromatography column and/or membrane adsorber, buffer volume, number of cycles, and process time. For example, with the increase of HCP, the size or volume of the AEX or CEX column may increase.

Further, the consumption amount(s) of one or more media necessary for the downstream processing of the biomass (such as in an anion exchange chromatography operation (AEX) or cation exchange chromatography operation (CEX) may be determined. Based on the determined consumption amount(s), it is possible to automatically control the stock of the respective media and to automatically order additional amounts and/or change the estimated amount(s) of media to be stocked. This considerably reduces the number of labor-intensive and error prone manual operations and the error rate. Further, there it has considerable advantages in terms of logistic and stock management.

The (functional) relationships or rules linking the upstream host cell protein (HCP) and/or DNA concentration and the at least one process parameter of an anion or a cation exchange chromatography operation may be stored in a database or other suitable storage unit and used to configure, setup and/or control the downstream process.

In an example, the at least one upstream process feature may include the type and/or amount of at least one contaminant. The determining of the at least one downstream process parameter may comprise determining, based on the received measurement value of the type and/or amount of the at least one contaminant, at least one downstream process parameter including one of a biomass dilution parameter, a cycle time and processing column size parameter.

The at least one upstream process feature may include for example the amount of antifoam and/or amount of block polymer(s). The determining of the at least one downstream process parameter may comprise determining, based on the received measurement value of the type and/or amount of antifoam or block polymer(s), at least one downstream process parameter including one of a biomass dilution parameter, a cycle time and processing column size parameter.

For example, the type and/or amount of at least one contaminant, such as the amount of antifoam and/or antiblock polymer in the upstream process may affect the viscosity and thus the process features or steps of the downstream process. For example, higher level of antifoam and/or antiblock polymer may require higher parameter settings in the downstream process, such as higher cycle time, processing column(s) size, etc. Further, the information of the amount of antifoam and/or antiblock polymer may be used to determine the level of dilution, wherein the higher the level of the antifoam and/or antiblock polymer in the upstream process, the higher the level of dilution in the downstream process.

Further, antifoam (AF) and antiblock polymer(s) may cause filter blocking. Accordingly, in a downstream process the amount of antifoam and/or antiblock polymer(s) may be kept as low as possible in the individual downstream process steps. This may be achieved for example by dilution and/or the use of a higher number of filters and/or filters with greater surface areas with increased amounts of antifoam and/or antiblock polymer(s).

The (functional) relationships or rules linking the upstream amounts and/or types of contaminants and the at least one downstream process parameter such as dilution, cycle time, processing time, etc. may be stored in a database or other suitable storage unit and used to configure, setup and/or control the downstream process.

In an example, the at least one upstream process feature may include the pH value and/or the amount of at least one pH correctant (puffer capacity). The determining of the at least one downstream process parameter may comprise determining, based on the received measurement value of the pH value and/or the amount of at least one pH correctant, the amount and/or type of at least one pH correctant for use in the downstream process. Generally, for a given process step, there is an optimal pH value or pH range. The higher the amount by which the measured pH value is greater than the optimal or target pH value, the more base/base concentration is needed and the respective downstream process settings can be set or configured accordingly. Analogously, the higher the amount by which measured pH value is lower than the optimal or target pH value, the more acid/acid concentration is needed and he respective downstream process setting may be set or configured accordingly.

For example, the pH value and/or the amount of at least one pH correctant (puffer capacity) of the upstream process may be used to determine the necessary or optimal amount of and/or type of pH correctant(s) for a protein A chromatography unit operation of the downstream process. In an example, a combination of the pH value (basic or ground pH value) and the pH correcant(s) may be used, in order to determine the necessary or optimal amount and/or type of pH correctant(s) for the downstream process.

Based on the determined amount and/or type of at least one pH correctant for the downstream process, the volume, flow rate, timing and other parameters of the delivery of the pH correctant(s) in the downstream process may be automatically setup, configured and/or controlled, for example by controlling the respective pumps and/or puffer reservoirs. Thus, the error rate may be reduced, the preparation time optimized and the process control simplified. Further, the stock management of pH correctant(s) may be improved.

Further, based on the pH value and/or the amount of at least one pH correctant (puffer capacity) of the upstream process, it may be determined whether to start or not start a downstream processing at all. For example, it the measured pH value is not within a certain threshold, the downstream process may be aborted, i.e. not started.

The (functional) relationships or rules linking the upstream pH value and the at least one downstream process parameter, such as the amount of at least one pH correctant, the amount and/or type of at least one pH correctant, etc. may be stored in a database or other suitable storage unit and used to configure, setup and/or control the downstream process.

Various other upstream process features may be measured and the measured values used to determine (e.g. set of change) at least one downstream process parameter:
the at least one upstream process feature may for example include the amount of lipids and/or long epidermal growth factor (EGF) and/or peptides and/or other media, and the determining of the at least one downstream process parameter may comprise determining, based on the received measurement value of the amount of lipids and/or long epidermal growth factor and/or peptides and/or other media, at least one clarification operation parameter or setting (such as for example the size and/or number and/or loading cycles, etc. of the chromatography columns); and/or
the at least one upstream process feature may include at least one critical quality attribute (CQA), and the determining of the at least one downstream process parameter may comprise determining, based on the received measurement value of the at least one critical quality attribute, whether to start a downstream process; and/or
the at least one upstream process feature may include the process temperature, and the determining of the at least one downstream process parameter may comprise determining, based on the received process temperature, a cooling or heating of process fluid for the downstream process; and/or
the at least one upstream characteristic may include turbidity, and the determining at least one downstream process parameter may comprise determining, based on the received measurement value of the turbidity, at least one clarification operation parameter or setting (such as for example the size and/or number and/or loading cycles, etc. of the chromatography columns, amount of dilution, etc.); and/or
the at least one upstream process feature may include media buffer system and/or capacity, and the determining of the at least one downstream process parameter may comprise determining, based on the received measurement value of the media buffer system and/or capacity, a pH adjustment parameter; and/or
the at least one upstream process feature may include process volume, and wherein the determining of the at least one downstream process parameter may comprise determining, based on the received measurement value of the process volume, dimensioning of the downstream process (the process volume is typically a global parameter that (in conjunction with other measured parameters) may be used to determine the settings and/or functionalities of one or more (for example all) downstream process steps); and/or
the at least one upstream feature may include conductivity, and wherein the determining of the at least one downstream process parameter may comprise determining, based on the received measurement value of the conductivity, a dilution parameter (for example based on the measured upstream conductivity and a predetermined optimal or target conductivity for at least one downstream process stage corresponding measures like amount of dilution to achieve the optimal or target level of conductivity for a given DSP process step may be determined).

The respective (functional) relationship between the measured at least one upstream feature and the at least one downstream parameter may be stored in a database or other suitable storage unit and used to configure, setup and/or control the downstream process.

According to a third aspect of the present disclosure, there is provided a computer program product comprising instructions which, when the program is executed by a computer, cause the computer to carry out the method of any the above described aspects and examples.

According to a fourth aspect of the present disclosure, there is provided a system configured to setup and/or configure and/or control a downstream process for processing a biomass to obtain a product. The system comprises at least one processor configured to carry out the method of any of the above described aspects and examples. Accordingly, the above description of embodiments, examples, technical effects and advantages in connection with the above described aspects and examples applies also to the system according to the fourth aspect of the disclosure.

In particular, the system may comprise a receiving device (which may be a part of the at least one processor of connected to the at least one processor) configured to receive at least one measurement value of at least one feature of an upstream process by which the biomass to be processed in the downstream process has been obtained. Further, the system is configured to determine, by the at least one processor, at least one downstream process parameter based on the received at least one measurement value.

Further, the system may be configured to configure and/or setup and/or control, by the at least one processor, at least one process stage or unit process operation of the downstream process based on the determined at least one downstream process parameter. The at least one processor may be a general or special purpose processor. It is also possible to employ a distributed computing system such as a cloud-based computing system. The system for configuring and/or setup and/or control of a downstream process for processing a biomass to obtain a product is also referred to as a downstream process setup and/or control system.

The system for setup and/or configuring and/or control of a downstream process may further comprise a data storage unit for storing the determined at least one at least one downstream process parameter. The data storage unit may be for example a database, such as the database described in connection with the first and the second aspects of the present disclosure.

The system for setup and/or configuring and/or control of a downstream process may further comprise one or more probes, sensors, or measuring devices to monitor one or more parameters of the downstream processes. The measurements may be carried out continuously or at specified intervals throughout the downstream process. The measurements may be used by the one or more control devices for controlling at least one piece of equipment associated with the downstream process (e.g. by using a feedback control loop).

Further, the measurements may be used to setup, configure and/or control at least one process parameter of the upstream process by which the biomass to be processed in the downstream process is obtained. In other words, the exchange of measurement data between the upstream and the downstream process may be bi-directional.

According to a fifth aspect of the present disclosure there is provided a method for processing a biomass comprising:
configuring and/or setup of a downstream process for processing the biomass according to the method of any one of the above described aspects and examples;
processing the biomass by using the so configured and/or setup downstream process.

The above description of embodiments, examples, technical effects and advantages in connection with the above described aspects applies also to the method according to the fifth aspect of the present disclosure.

The method may further comprise
performing an upstream process thereby obtaining the biomass to be processed in the downstream process; and
measuring at least one feature of the upstream process.

As described above in connection with the first and the second aspect, the measuring of the at least one feature of the upstream process may be performed performed online (e.g. inline or atline) or offline.

According to a sixth aspect of the present disclosure, there is provided a system configured to process a biomass, said system comprising:
at least one processor configured to perform a method for configuring and/or setup and/or control of a downstream process for processing the biomass according to any one of the above aspects and examples;
at least one processing stage configured to process the biomass by using the so configured and/or setup downstream process.

As described above in connection with the first and the second aspect, the at least one processing stage may be a filter stage, chromatography stage, stirring stage, hold-up stage, or any other suitable stage. The at least one processing stage may comprise respective pieces of equipment for performing the one or more process operations associated with the respective processing stage and one or more control devices for controlling the equipment associated with the respective processing stage.

The system for processing biomass may further comprise one or more probes, sensors, or measuring devices to monitor one or more parameters of the downstream processes in at least one process stage. The measurements may be carried out continuously or at specified intervals throughout the process. The measurements may be used by the one or more control devices for controlling at least one processing equipment associated with the respective processing stage (e.g. by using a feedback control loop). The measuring of the at least one process parameter of the downstream process may be performed inline, online, atline, or offline.

The system may further comprise
at least one processing stage configured to carry out an upstream process to thereby obtain the biomass to be processed in the downstream process;
at least one measuring device for measuring of at least one feature of the upstream process.

As described above in connection with the first and the second aspect, the measuring of the at least one feature of the upstream process may be performed online (e.g. inline or atline) or offline. Further, the above description of embodiments, examples, technical effects and advantages in connection with the above described aspects applies also to the system according to the sixth aspect of the disclosure.

The above described principles, methods and devices may also be employed not only to integrate upstream and downstream processes but also to integrate different sub-processes and/or different devices, systems or units within one type of process, for example within an upstream process or a downstream process.

For example, it is possible to use measurement data/values related to at least one feature or parameter in one particular process stage, device and/or unit to configure, set and/or control another process stage, device or unit. The process stages may be, for example, cell growing stage, cell harvest stage, stirring stage, media preparation stage, buffer exchange stage, perfusion stage, kSep, bag testing stage, chromatography stage, filtering stage, etc.

For example, it is possible to use measurement data/values obtained in a chromatography stage and more specifically in one run of the chromatography stage or operation to optimally configure and/or set (i.e. to parametrize) and/or to control a subsequent process stage or operation, such as a virus activating. In such applications, the cyclic operation of a chromatography stage may be challenging from process control perspective. This may be alleviated by the proposed method.

The exchange of data may be bi-directional. Thus, it is possible to use measurement data of a second process stage following a first process stage to configure, set and/or control the first process stage.

Thus, according to a seventh aspect of the present disclosure, there is provided a computer-implemented method for configuring and/or setup and/or control of a process for processing a biomass to obtain a product comprising a plurality of process operations or stages, comprising:
receiving at least one measurement value of at least one feature of a first one of the plurality of process stages or operations;
determining, based on the received at least one measurement value, at least one process parameter of a second one of the plurality of process stages or operations.

According to an eighth aspect of the present disclosure, there is provided a computer program product comprising instructions which, when the program is executed by a computer, cause the computer to carry out the method of the seventh aspect.

According to a ninth aspect of the present disclosure, there is provided a system configured for configuring and/or setup and/or control of a process for processing a biomass to obtain a product. The system comprises at least one processor configured to carry out the method according to the seventh aspect.

According to a tenth aspect of the present disclosure there is provided a method for processing a biomass to obtain a product comprising:
configuring and/or setup and/or controlling of a process for processing the biomass according to the method of the seventh aspect of the present disclosure;
processing the biomass by using the so configured and/or setup and/or controlled downstream process.

According to an eleventh aspect of the present disclosure there is provided a system configured to process a biomass to obtain a product, said system comprising:
at least one processor configured to perform a method for configuring and/or setup and/or control of a process for processing the biomass according to the seventh aspect of the present disclosure;
at least one processing stage configured to process the biomass by using the so configured and/or setup process.

Further, the above description of embodiments, examples, technical effects and advantages in connection with the above described first to sixth aspects and examples applies also to the nth seventh to eleventh aspects of the present disclosure.

Advantages of an exchange of data (uni-directional or bi-directional) between different process stages, device and/or units or between an upstream and a downstream process include but are not limited to:
- optimal time synchronization of the processes in the different process stages, devices and/or units or between the upstream and downstream processes;
- reduction of idle, non-productive periods and increasing of the process efficiency;
- improving of the yield and/or quality of the final products;
- reducing the waste;
- optimizing media consumption and/or storage;
- optimizing the inventory management and logistic.

These and other aspects will now be described in detail with reference to the following drawings:
**Fig. 1** showing schematically an exemplary system for configuring and/or setup of a downstream process for processing a biomass to obtain a final product;
**Fig. 2** showing schematically another exemplary system for processing a biomass.

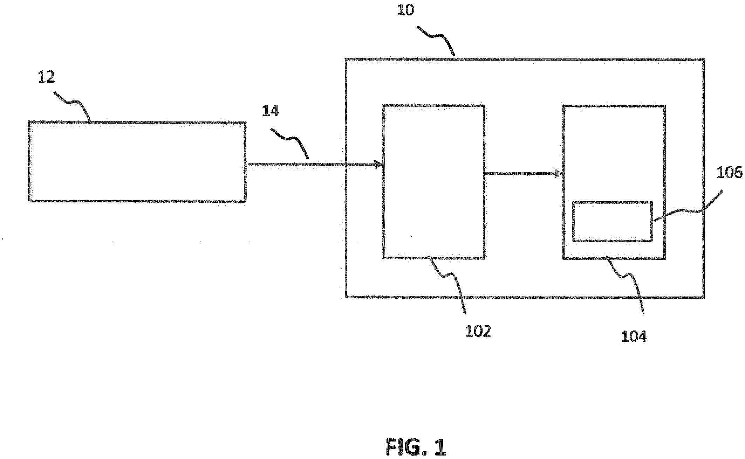

**Fig. 1** shows schematically an exemplary system 10 for configuring and/or setup and/or control of a downstream process for processing a biomass to obtain a final product. The system 10 comprises a receiving device 102 for receiving measurement value(s) 12 of at least one feature of an upstream process (upstream feature) by which a biomass to be processed by the downstream process is obtained. The measurement value(s) 12 of the at least one upstream feature may be transmitted to the receiving device 102 over a a suitable communication network 14, such as a computer network, wirelessly or by any other suitable communication means. The measurement value(s) 12 of the at least one upstream feature may be measured and/or transmitted online or offline.

Exemplary online upstream features may include any of the following features:
- pH (used for example to set/control a pH parameter of a downstream process);
- conductivity (used for example to set/control a conductivity parameter of a donwstream process),
- temperature (used for example to set/control a temperature parameter of a donwstream process),
- culture volume (used for example to configure/set/control a downstream process, such as diatomeaceos earth cell harvest process),
- antifoam concentration (used for example to configure/set/control a downstream process, for example a high antifoam concentration may be negative for the downstream process),
- cell density, cell culture turbidity, cell viability (used for example to configure/set/control a downstream process, such as diatomeacoes earth process, set the clarification settings, such as for example centrifugal settings, filter settings, etc.),
- flow sensor parameter, including sensor quality (used for example to configure/set/control a continuous downstream process, such as a continuous downstream process from a perfusion culture),
- spectroscopy data, e.g. indicative of the total protein concentration,
- metabolite and/or medium concentration;
- pressure value of a tube and/or bioreactor, etc.

Exemplary offline upstream features may include any of the following features:
- cell viability;
- total protein concentration obtained by offline measurement or offline sample;
- lactate dehydrogenase or other enzyme activity;
- upstream product quality, e.g. of a monoclonal antibody;
- upstream product aggregation level;
- product concentration;
- DNA/HCP concentration.

The system 10 comprises further at least one processor 104 configured to determine at least one downstream process parameter based on the received at least one measurement value. The at least one processor 104 is further configured to automatically set or configure and/or control at least one process stage or process operation of the downstream process based on the determined at least one downstream process parameter. The processor 104 further comprises a database 106 for storing the measured values of one or more upstream features and/or predetermined (functional) relationships or rules that link the one or more upstream features with one or more downstream features. The database 106 may be a standalone unit that is linked to the at least one processor 104 via suitable communication link, for example over the internet, WLAN, etc.

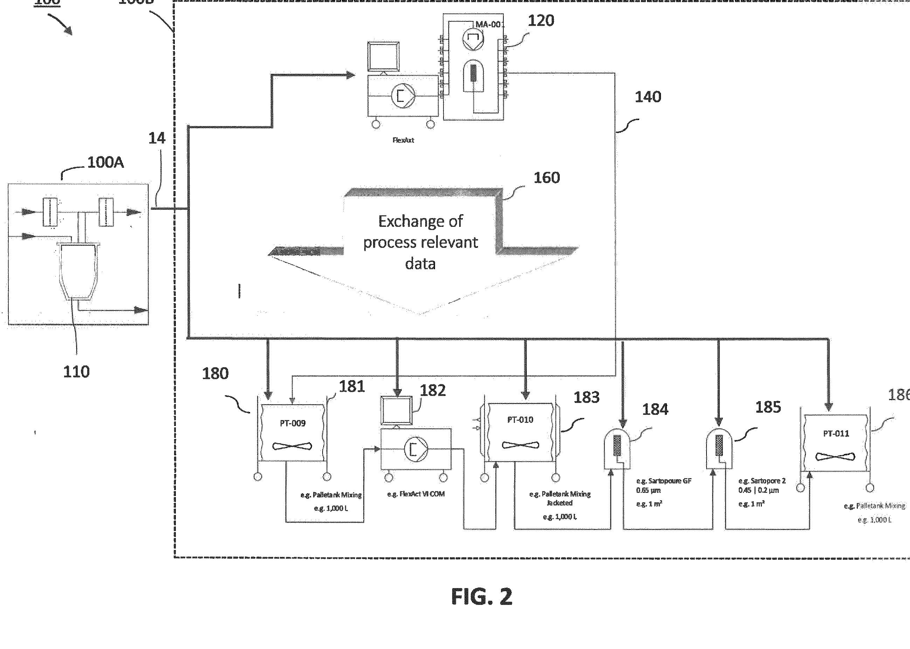

**Fig. 2** shows schematically an exemplary system 100 for processing a biomass to obtain a product comprising a plurality of processing stages. The system 100 comprises an upstream processing sub-system 100A and a downstream processing sub-system 100B.

The upstream processing sub-system 100A includes at least one bioreactor 110. The bioreactor 110 may be of any scale, from a small lab-size bioreactor to a very large bioreactor with volumes of about 2000 L and higher. Bioreactors of various sizes are available for example from Sartorius Stedim Biotech. Measurement value(s) of at least one upstream feature of the upstream process(es) carried out by the upstream processing sub-system 100A are transmitted to the downstream processing sub-system 100B over a communication network 14, such as over a computer network, wirelessly or by any other suitable communication means. The transmitted measurement values of the at least one upstream feature may be used to configure or setup the downstream process carried out by the downstream processing sub-system 100B.

The downstream processing sub-system 100B comprises a plurality of processing stages. The number, type, size, etc. of the processing stages may vary, depending on the specific application. As shown in Fig. 2, one or more of the processing stages of the downstream processing sub-system 100B may be configured or setup based on the measurement data received from the upstream processing sub-system 100A.

In the example shown in Fig. 2, the e downstream processing sub-system 100B comprises a Protein A chromatography stage 120 followed by a virus inactivation stage 180. The virus inactivation stage 180 comprises a plurality of sub-stages 181-186, each performing a particular sub-process or group of sub-processes. The sub-stage 181 is a stirring stage, e.g. a stirring stage in a 1000 L Palletank^{®} of Sartorius Stedim Biotech. The sub-stage 182 is a low pH virus inactivating stage, such as for example FlexAct^{®} VI of Sartorius Stedim Biotech. The sub-stage 183 is a stirring stage, e.g. in a 1000 L Palletank^{®} of Sartorius Stedim Biotech. The sub-stages 184 and 185 are filtering stages, e.g. by using a Sartopure^{®} GF 0.65 µm filtering unit, the sub-stage 186 is a is a stirring stage, e.g. a stirring stage in a 1000 L Palletank^{®} of Sartorius Stedim Biotech . The processing stages shown in Fig. 2 are only exemplary. As mentioned above, it is possible to use other stages or stage combinations, such as other stages of other types, sizes, etc.

Measurement values of at least one feature of the process(es) carried out by one or more of the stages of the downstream processing sub-system 100B (downstream feature) may be obtained and transmitted to other stages of the downstream processing sub-system 100B over a communication network 140, such as a computer network. The transmitted measurement values of the at least one downstream feature may be also used to configure or setup the at least one other stage to which these values are transmitted. The measurement values of at least one upstream feature and/or the measurement values of at least one downstream feature form a part of process relevant data 160 exchanged within the downstream processing sub-system 100B and used to configure or setup and/or control one or more of the downstream processing stages. The exchange of process relevant data may be, for example, managed by a respectfully configured processing unit.

For example, the exchanged process relevant data 160 may comprise one or more of features of the Protein A chromatography stage 120 that may be measured and transmitted over a communication network (e.g. a computer network) 140 to the virus inactivation stage 180 and/or other downstream stages. The one or more features of the Protein A chromatography stage 120 may include the number of chromatography cycles, progress in time, volume of one chromatography cycle, puffer strength or amount, type of puffer, pH of the eluate, etc. Based on the received feature(s), one or more process parameters of the inactivation stage and/or other downstream stages are configured or setup.

Below are some examples of process parameter setup or configuration based on measured values of upstream and/or downstream features using for example the systems shown in Figs. 1 and 2.

**Table 1** shows exemplary upstream process (USP) features and the respective downstream process (DSP) parameters that may be set/configured or adjusted. In Table 1, category 1 denotes cells or cell cultures, category 2 denotes products, category 3 denotes impurities and category 4 denotes process control parameter. Different levels of priority may be attributed to the individual measured USP features depending on their importance, for example 1: high priority, 2: medium priority and 3: low priority.

| **Priority** | **Category** | **On-/ Offline** | **USP Parameter** | **Utilization in DSP** | **Description** |
|---|---|---|---|---|---|
| 1 | 1 | Online or offline | Viable cell density (VCD), Biomass, Capacitance | Clarification settings (e.g. filter area, type, amount of diatomaceous earth, etc.) | VCD may be multiplied with the process volume to obtain the total amount of biomass. Based on the total amount of biomass, the amount of the cell mass that needs to be separated may be determined. Based on the determined amount of the cell mass that needs to be separated and predetermined settings (stored for example in a database), the preferred method/system for the cell separation step may be selected. For example, with the increase of the cell mass the following separation methods may be selected in this order: Deep Filtration, Dynamic Body Feed Filtration, Centrifugation. In addition, the number of filters and/or filter area may be set/configured. |
| | | | | | Further, based on the upstream VCD, biomass and/or capacity the amount of dilution of the biomass to be processed in the downstream process may be determined and the downstream process accordingly setup, configured and/or controlled. |
| 1 | 1 | Offline | Viability | Clarification settings (e.g. filter area, type, amount of diatomaceous earth, etc.), centrifugation g-force | See e.g. Table 3 below |
| 1 | 2 | Online or offline | Titer | Dimensioning, loading of capture, loading volume, cycle time and number, etc. | 1. Batch-Prozess: The total product amount that is to be purified or cleared in a DSP process (for example by Protein A Chromatography) may be determined by multiplication of the titer and the process volume. Based on the total amount of product, the chromatography process parameters such as the dimensioning of the chromatography column(s) to complete the process within a predetermined time may be determined. Alternatively, the process time (and thus the number of cycles) and/or the puffer amount for a predetermined column dimensioning/size may be determined and the chromatography process accordingly configured. |
| | | | | | 2. Continuous Prozess: Based on the titer and the volume flow/volume flow rate the number and/or dimensioning of chromatography columns necessary to ensure continuous flow and processing from the USP to the DSP may be determined. Further or alternatively, the number of cycles, the timing of the column exchange (e.g. due to the aging of the columns after a certain number of cycles) may be determined. Still further, it is possible to generate and/or adjust the recipe based on the measured USP titer (e.g. by setting or adjusting the loading volume, the volume flow rate, etc.). See also e.g. Table 2 below |
| 3 | 3 | Offline | Host Cell Protein (HCP) | Dimensioning of AEX/CEX | Generally, the higher the amount of HCP, the larger the size of the AEX/CEX column. |
| 3 | 3 | Offline | DNA | Dimensioning of AEX/CEX | Based om the DNA concentration the dimensioning of the anion exchange chromatography operation (AEX) or the cation exchange chromatography operation (CEX), such as for example the volume of the column and/or the membrane adsorber, number of cycles, process time, etc., may be determined. Further, the determined number and/or amount of consumed media and/or equipment may be automatically compared with available/stocked media and/or equipment and, if needed, additional media and/or equipment ordered. |
| 3 | 3 | Online | Amount of antifoam | Corresponding counter measures like dilution, number of filters, etc. | Depending on the amount of antifoam (AF), a dilution parameter and/or the filter setting (e.g. number of filters) may be determined and set, in order to keep the amount of antifoam as low as possible in the downstream process steps. |
| 3 | 3 | Offline | Amount and/or type of lipids | Dimensioning of the chromatography column(s) | Generally, the more lipids, the greater the size and/or number and/or loading cycles, etc. of the chromatography columns. |
| 3 | | Offline | Amount and/or type of block polymers | Corresponding counter measures like dilution and/or increasing the number of filters | Depending on the amount of block polymer(s), a dilution parameter and/or the filter setting (e.g. number of filters) may be determined and set, in order to keep the amount of block polymer(s) as low as possible in the downstream process steps. |
| 3 | 4 | Online 1 | Amount and/or type of pH correctants (acid/base) | Correction to optimal pH/binding conditions | A combination from the pH value (basic-pH-value) und pH correctants (puffer capacity) may be used to determine the amount and/or type of pH correctants that are necessary, in order to optimally set/configure the pH value of the DSP process steps or stages (such as (Protein A Chromatography). |
| | | | | | See also e.g. Table 5 below |
| 2 | 4 | Online | pH value | Correction to optimal pH/binding conditions | A combination from the pH value (basic-pH-value) und pH correctants (puffer capacity) may be used to determine the amount and/or type of pH correctants that are necessary, in order to optimally set/configure the pH value of the DSP process steps or stages (such as (Protein A Chromatography). |
| | | | | | See also e.g. Table 5 below |
| 2 | | Online | Conductivity | Corresponding counter measures like dilution | Corresponding counter measures like dilution to set an optimal or target level of conductivity for a given DSP process step. The feature "conductivity" may be also be measured for a given downstream process step and used to configure a subsequent downstream process step. |
| 1 | 2 | Offline | Critical Quality Attribute (CQA) | Go/noGo for DSP | If critical quality attribute(s) is/are not fulfilled, the downstream process is terminated (no Go). If critical quality attribute(s) is/are fulfilled, the downstream process starts or continues (Go). |
| 3 | 3 | Offline | Other media / process components (long EGF, peptides, etc.) | Dimensioning of the chromatography column(s) | Generally, the more other media or process components (such as long EGF, peptides, etc.), the greater the size and/or number and/or loading cycles, etc. of the chromatography columns. |
| 1 | 4 | Online | Process temperature | Optimizing cooling/heating of process fluid for DSP | Configuring the temperature settings. |
| | | | | | see e.g. Table 6 below |
| 1 | 3 | Online or offline | Turbidity | clarification settings (filter area, type, etc.) | The measured turbidity may be an indicator of the amount of biomass (e.g. cell mass) that needs to be separated in a downstream process. Based on the amount of biomass that needs to be separated and predetermined settings (stored for example in a database), the preferred method/system for the cell separation step may be selected. For example, with the increase of the cell mass the following separation methods may be selected in this order: Deep Filtration, Dynamic Body Feed Filtration, Centrifugation. In addition, the number of filters and/or filter area may be set/configured. |
| | | | | | Further, based on the measured turbidity, the amount of dilution of the biomass to be processed in the downstream process may be determined and the downstream process accordingly setup, configured and/or controlled. |
| 3 | 4 | Offline | Media buffer system and capacity | pH adjustment in DSP | Adjustment of the pH settings in the downstream process |
| 1 | 4 | Online or offline | Process volume | Dimensioning of DSP, etc. | The process volume is a global parameter that may be used when determining one or more other parameters as explained above. Thus, the process volume may be used to determine the settings and/or functionalities of one or more (for example all) downstream process steps. |

Not all of the USP features listed in Table 1 need to be taken into account when configuring the DSP process. For example, only USP features with high priority or USP features with high and medium priority may be considered. In general, the configuration of at least one DPS process stage or operation is carried out automatically based on at least one of the upstream features listed in Table 1.

Below are some further examples of downstream process configuration based on upstream features.

The measured upstream feature may be for example titer. A high titer multiplied by the process volume results in a large quantity of product. Generally, the higher the titer, the smaller the volume that is laded in/over a chromatography column in a loading step.

Accordingly, with the increase of the titer in a USP process, in a DSP process, a larger size and/or higher number of chromatography columns and/or a higher number of cycles may be set/configured. Further, the column exchange sequence, loading scenario and/or sequence can be appropriately configured.

**Table 2** shows exemplary setup/configuration of the downstream process (DSP) based on the measured titer during the upstream process (USP).

**Table 2**

| **Upstream Process** | **USP Feature(s)** | **DSP Process parameters** |
|---|---|---|
| 50 L process with 5 g/L product (Fed-Batch toward the process end) | 250 g product | Example: 1,5 L Protein A chromatography column, 6 cycles |
| 50 L process with 10 g/L product (Fed-Batch toward the process end) | 500 g product | Example: 3L Protein A chromatography column, 6 cycles (in case of fast processing) or 1,5L Protein A chromatography column with 12 cycles (in case of slower but more cost-effective process) |
| Perfusionprocess with 0.5 - 4 vvd (25 - 200L per day) with constant product concentrations of 1 g/L | 25 - 200 g product over 24 hours | DSP process parameters that may be set/configured include for example one or more of: chromatography column size and/or number, cycles. |
| Perfusionprocess with 0,5 - 4 vvd (25 - 200L per day) with fluctuating product concentration | Fluctuating product amount over 24 hours | DSP process parameters that may be set/configured include one or more of: chromatography column size and/or number, cycles, column exchange sequence/scenario (e.g. one column being loaded, one being used for purification according to a column backup principle), adaptive column selection and loading scenario/sequence depending on the process characteristics |

Additionally or alternatively the measured upstream feature may be cell viability. **Table 3** shows exemplary setup/configuration of the downstream process based on the measured upstream cell viability. Generally, based on the measured/target cell viability, the clarification operation settings (such as, for example, the g-force of the centrifugation stage) may be determined. As a general rule, the higher the targeted viability at the end of the centrifugation, the lower the g-force of the centrifugation stage.

In addition or alternatively, the clarification settings (such as for example the g-force of the centrifugation stage, duration of centrifugation, etc.) may be selected based on the targeted degree of sedimentation. For example, the higher the targeted degree of sedimentation (independent of the cell viability), the higher the g-force and/or the longer the duration of centrifugation.

**Table 3**

| **USP Feature(s)** | **DSP Process parameters** |
|---|---|
| Transfer of the measured viability | Depending on the target requirements for the cell viability a g-force of the centrifugal stage/operation may be set/configured, e.g. a low g-force range (e.g. up to 300 N x g), a middle range g-foce (e.g. from 300 N x g to 3000 N x g) or a high g-force range (e.g. higher than 3000 N x g) . For example a centrifugal stage/operation with a low g-force (e.g. up to 300 N x g) may be selected for cells that should retain high viability. The middle range g-force may be selected in case there is no need to retain high target cell viability and the high range g-force may be selected in case of strong sedimentation and/or for centrifugal efficiency in case of damaged (apoptotic) cells. |

Additionally or alternatively the measured upstream feature may be (viable) cell density or concentration. Generally, the higher the measured (viable) cell density or concentration, the greater the filter area and/or the number of filters and/or the number of filter cycles, etc. This also applies regarding the measured biomass: the larger the amount of measured biomass, the greater the filter area and/or the number of filters and/or the number of filter cycles, etc.

**Tables 4A** and **4B** show exemplary setups/configurations of the downstream process based on the measured upstream cell density or concentration. In particular, Table 4A shows exemplary selection of the downstream cell separation method for CHO cells based on the measured upstream cell density or cell concentration. Table 4B shows exemplary configuration/setup of the employed filter for CHO cell separation based on the measured upstream cell density or cell concentration.

**Table 4A**

| **USP feature(s)** | | **DSP processing parameter** |
|---|---|---|
| *Viable cell density (VCD) [* 10^6*/*ml]* | *Moist Biomass [g*/*l]* | *Cell separation method* |
| < 10 | < 50 | Deep filtration + Sterile filtration |
| 10 - 25 | 50 - 150 | Dynamic Body Feed Filtration + Sterile filtration |
| > 25 | > 150 | Centrifugation + Deep filtration + Sterile Filtration |

**Table 4B**

| **USP Feature(s)** | | **DSP Process parameters** | | | | |
|---|---|---|---|---|---|---|
| *Volume [l]* | *Moist biomass [g*/*l]* | *Separation method* | *Filter Surface area [m²]* | *Filter Specification* | *Sterile filter surface area [m²]* | *Sterile filter Specification* |
| 200 | 50 | 2-stage Deep Filtration - Sterile Filtration | 6,4 | 1. Stage: 5x Sartoclear DL90 Cassettes; | 1,6 | Sartopore2 XLG, Size 2 |
| | | | | 2. Stage: 3x Sartoclear DL20 Cassettes | | |
| 200 | 80 | Dynamic Body Feed + Sterile Filtration | 0,63 | 3x Sartoclear Dynamic Cassettes + 4,5 kg Kieselgur (diatomaceous earth) | 0,8 | Sartopore2 XLG, Size 1 |
| 500 | 80 | Dynamic Body Feed + Sterile Filtration | 1,47 | 7x Sartoclear Dynamic Cassettes + 10,5 kg Kieselgur (diatomaceous earth) | 2,4 | Sartopore2 XLG, Size 3 |
| 500 | 120 | Dynamic Body Feed + Sterile Filtration | 2,1 | 10x Sartoclear Dynamic Cassettes + 15 kg Kieselgur (diatomaceous earth) | 2,4 | Sartopore2 XLG, Size 3 |

Additionally or alternatively the measured upstream feature may be pH. **Table 5** shows exemplary configuration/setup of the downstream process based on the measured upstream pH. The pH configuration/setup depends on the pH requirements (target pH) of the specific process stage. If the pH is within the targeted range, no further adjustment is needed. If the pH deviates from the targeted range, an addition of pH correctants (acid or base type) may be required. The higher the deviation of the pH from the targeted pH, the higher the amount of pH correctants.

**Table 5**

| **Downstream Process** | **USP Feature** | **DSP Process parameter(s)** |
|---|---|---|
| Antibodyprocessing | pH in the range of 6.8 to 7.1 | Carrying over the USP pH value to the DSP process. Generally, no further adjustment of the DSP processs is needed, since the binding capacity is good for this pH range |
| Cell separation (e.g. Kieselgurfiltration with pH Shift) | pH in the range of 6.8 to 7.1 | Increasing acidity to reach a pH in the range of 5.0 -to 5.5. pH. Based on the USP pH value, it is possible to determine the acid amount and/or concentration necessary to reach the target DSP pH value depending on the DSP process (e.g. depending on the puffer capacity of the medium) and set/configure the DSP process accordingly. |

Additionally or alternatively the measured upstream feature may be temperature. **Table 6** shows exemplary configuration/setup of the downstream process based on the measured upstream temperature.

**Table 6**

| **Downstream Process [Correct?]** | **USP Feature(s)** | **DSP Process parameters** |
|---|---|---|
| Temperature of the culture medium | Temperature of the bioreactor (at the end of the process or at the subsequent cooling step, e.g. from 37°C to 5°C | Pre-heating or pre-cooling of the receiving tank, in particular in case of temperature instable products and/or in order to avoid interactions in case of puffers. |

The settings, including the (functional) relationships or rules linking the upstream features and downstream parameters in the above Tables 1 to 6 may be stored in the database 106 and used to setup, configure and/or control the downstream process.

At least one of the upstream features listed in the above Tables 1 to 6 may also be measured in a particular stage of the downstream process and used to set of configure a subsequent stage or stages of the downstream process. One exemplary feature that may be measured in a downstream process stage and used to configure a subsequent downstream process step is conductivity. Other exemplary features are pH, pressure, temperature, cell density, turbidity, cell viability, flow rate, metabolite and/or medium concentration, contaminant's concentration, protein concentration, etc.

As described above various other upstream features may be measured and used alone or in combination to setup/configure and/or control the downstream process.

The computational aspects described here can be implemented in digital electronic circuitry, or in computer hardware, firmware, software, or in combinations of them. When appropriate, aspects of these systems and techniques can be implemented in a computer program product, for example tangibly embodied in a machine-readable storage device for execution by a programmable processor; and method steps can be performed by a programmable processor executing a program of instructions to perform functions by operating on input data and generating an output.

To provide for interaction with a user, a computer system can be used having a display device, such as a monitor or a LCD screen for displaying information to the user and a keyboard, a pointing device such as a mouse or a trackball, a touch-sensitive screen, or any other device by which the user may provide input to computer system. The computer system can be programmed to provide a graphical user interface through which the computer program(s) interact(s) with the user.

A number of embodiments and examples have been described. Nevertheless, it will be understood that various modifications may be made. For example, the steps described can be performed in a different order and still achieve desirable results. Further, individual features of different embodiments and examples may be combined. Accordingly, other embodiments are within the scope of the claims.

Further, unless otherwise defined, all terms (including technical and scientific terms) used herein have the same meaning as commonly understood by one of ordinary skill in the art to which this invention belongs.

### List of Reference Numerals

10 system for configuring and/or setup and/or control of a downstream process for processing a biomass
102 receiving device
104 process/processor system
106 database
12 upstream measurement values
14 communication network (e.g. computer network)
100 system for processing a biomass
100A upstream processing sub-system;
100B downstream processing sub-system;
110 bioreactor
120 protein A chromatography stage
140 communication network (e.g. computer network)
160 process relevant data
180 virus inactivation stage
181 stirring stage
182 low pH virus inactivating stage
183 stirring stage
184, 185 filtering stage
186 stirring stage

## Claims

1. A computer-implemented method for configuring and/or setup and/or controlling of a downstream process for processing a biomass, comprising:
receiving at least one measurement value of at least one feature of an upstream process by which the biomass to be processed in the downstream process has been obtained;
determining at least one downstream process parameter based on the received at least one measurement value.

2. The method according to claim 1, wherein the at least one upstream process feature includes a viable cell density, biomass, capacitance, cell viability and/or turbidity and wherein the determining of the at least one downstream process parameter comprises:
selecting a clarification operation;
determining, based on the received at least one measurement value of the viable cell density, biomass, capacitance and/or cell viability, at least one process parameter of the selected clarification operation and/or determining a biomass dilution parameter.

3. The method according to claim 2, wherein:
wherein selecting a clarification operation includes selecting between a filter operation and a centrifugal operation, wherein:
if a filter operation is selected, the at least one process parameter includes at least one of the following parameters: a type of the filter operation, number of filters, filter area, pressure, flow rate amount of diatomaceous earth and process time;
if the centrifugal operation is selected, the at least one process parameter includes the process time, process volume and centrifugal force.

4. The method according to any one of the preceding claims, wherein the at least one upstream process feature includes titer, and wherein the determining of the at least one downstream process parameter comprises determining, based on the received measurement value of the titer, at least one process parameter of a chromatography operation, said at least one process parameter including one or more of a dimensioning, loading volume, cycle time, flow rate and number of chromatography columns.

5. The method according to any one of the preceding claims, wherein the at least one upstream process feature includes a host cell protein and/or DNA concentration and wherein the determining of the at least one process parameter of an anion or a cation exchange chromatography operation, said at least one process parameter including one or more of a volume of the chromatography column and/or membrane adsorber, buffer volume, number of cycles, and process time.

6. The method according to any one of the preceding claims, wherein the at least one upstream process feature includes the type and/or amount of at least one contaminant, and wherein the determining of the at least one downstream process parameter comprises determining, based on the received measurement value of the type and/or amount of at least one contaminant, a biomass dilution parameter, cycle time and/or processing column size.

7. The method according to claim 6, wherein the at least one upstream process feature includes the amount of antifoam and/or amount of block polymers.

8. The method according to any one of the preceding claims, wherein the at least one upstream process feature includes the pH value and/or the amount of at least one pH correctant, and wherein the determining of the at least one downstream process parameter comprises determining, based on the received measurement value of the pH value and/or the amount of at least one pH correctant, the amount and/or type of at least one pH correctant for use in the downstream process.

9. The method according to any one of the preceding claims,
wherein the at least one upstream process feature includes the amount of lipids and/or long epidermal growth factor, and/or peptides and/or other media, and wherein the determining of the at least one downstream process parameter comprises determining, based on the received measurement value of the amount of lipids and/or long epidermal growth factor and/or peptides and/or other media, at least one clarification operation parameter; and/or
wherein the at least one upstream process feature includes at least one critical quality attribute, and wherein the determining of the at least one downstream process parameter comprises determining, based on the received measurement value of the at least one critical quality attribute, whether to start a downstream process; and/or
wherein the at least one upstream process feature includes the process temperature, and wherein the determining of the at least one downstream process parameter comprises determining, based on the received process temperature, a cooling or heating of process fluid for the downstream process; and/or
wherein the at least one upstream characteristic includes turbidity, and wherein the determining at least one downstream process parameter comprises determining, based on the received measurement value of the turbidity, at least one clarification operation parameter; and/or
wherein the at least one upstream process feature includes media buffer system and/or capacity, and wherein the determining of the at least one downstream process parameter comprises determining, based on the received measurement value of the media buffer system and/or capacity, a pH adjustment parameter; and/or
wherein the at least one upstream process feature includes process volume, and wherein the determining of the at least one downstream process parameter comprises determining, based on the received measurement value of the process volume, dimensioning of the downstream process.

10. A computer program product comprising instructions which, when the program is executed by a computer, cause the computer to carry out the method of any one of the preceding claims.

11. A system (10) configured to configure and/or setup and/or control a downstream process for processing a biomass to obtain a product, said system comprising at least one processor (104) configured to carry out the method of any one of claims 1 to 9.

12. A method for processing a biomass comprising:
configuring and/or setup of a downstream process for processing the biomass according to the method of any one of claims 1 to 9;
processing the biomass by using the so configured and/or setup downstream process.

13. The method according to claim 12, further comprising:
performing an upstream process thereby obtaining the biomass to be processed in the downstream process;
measuring at least one feature of the upstream process.

14. A system (100) configured to process a biomass, said system comprising:
at least one processor (104) configured to perform a method for configuring and/or setup of a downstream process for processing the biomass according to any one of claims 1 to 9;
at least one processing stage (12; 18; 181-186) configured to process the biomass by using the so configured and/or setup downstream process.

15. The system (100) of claim 14, further comprising
at least one processing stage configured to carry out an upstream process to thereby obtain the biomass to be processed in the downstream process;
at least one measuring device for online or offline measuring of at least one feature of the upstream process.
